# EUROPÄISCHE PATENTSCHRIFT

(11) **EP 0 863 130 B1**
(45) Veröffentlichungstag und Bekanntmachung des Hinweises auf die Patenterteilung: **05.06.2002**
(21) Anmeldenummer: 98103119.8
(22) Anmeldetag: 23.02.1998
(51) Int. Cl.: C07C 209/36, C07C 211/55

(54) **Verfahren zur Herstellung von 4-Aminodiphenylamin**
Process for the preparation of 4-aminodiphenylamine
Procédé pour la préparation de 4-aminodiphénylamine

(30) Priorität: 06.03.1997 DE 19709124
(43) Veröffentlichungstag der Anmeldung: 09.09.1998
(73) Patentinhaber: BAYER AG, 51368 Leverkusen (DE)
(72) Erfinder: Buysch, Hans-Josef, Dr., 47809 Krefeld (DE); Laue, Christian, Dr., 40789 Monheim (DE); Königshofen, Heinrich, Dr., 51465 Bergisch Gladbach (DE); Notheis, Ulrich, Dr., 41539 Dormagen (DE); Ooms, Pieter, Dr., 47800 Krefeld (DE); Pentling, Ursula, Dr., 47239 Duisburg (DE)

(56) Entgegenhaltungen:
- US-A- 5 420 354

## Beschreibung

Die Erfindung betrifft ein Verfahren zur Herstellung von 4-Aminodiphenylamin (4-ADPA) durch Hydrierung von Nitrobenzol mit Wasserstoff in Anwesenheit geeigneter Hydrierkatalysatoren und geeigneter Basen.

4-ADPA ist ein wichtiges Zwischenprodukt für Alterungsschutzmittel und Stabilisatoren in der Gummi- und Polymerindustrie (Kirk-Othmer, Encyclopedia of Chemical Technology, 4th Edition, 1992, Vol. 3, S. 424-447 und S 448-456; Ullmann's Encyclopedia of Industrial Chemistry, 5th Edition, Vol A3, 1985, S 91-111).

4-Aminodiphenylamin kann nach verschiedenen Methoden hergestellt werden. Eine Möglichkeit 4-ADPA herzustellen, ist die zweistufige (Zwischenprodukt 4-Nitrodiphenylamin) Umsetzung von Anilin bzw. Anilinderivaten mit p-Nitrochlorbenzol in Gegenwart eines Säureakzeptors oder eines Neutralisierungsmittels und gegebenenfalls in Gegenwart eines Katalysators. Die Herstellung nach dieser Methode ist beispielsweise beschrieben in DE-A 35 01 698, DE-A 18 56 63, US 4 670 595, US 4 187 249, US 468 333 und US 4 187 248. Ein Nachteil eines solchen Verfahrens ist, daß die dabei entstehenden Halogenidionen zu Korrosion in Reaktoren und Apparaturen führen und mit beträchtlichen Kosten entsorgt werden müssen. Außerdem müssen die Ausgangsmaterialien, wie p-Chlornitrobenzol und gegebenenfalls die entsprechenden Formanilidderivate, in zusätzlichen Reaktionsschritten hergestellt werden.

Um solche Nachteile zu vermeiden, hat man Anilin bzw. entsprechende Anilinderivate mit Nitrobenzol in Gegenwart von Tetraalkylammoniumhydroxiden und in Gegenwart kontrollierter Mengen an protischen Materialien umgesetzt. 4-ADPA wurde dabei in einer befriedigenden Menge erhalten (siehe WO 93/00 324 und WO 93 24 450). Nachteilig bei diesen Verfahren ist jedoch, daß zwei verschiedene Einsatzprodukt eingesetzt werden müssen und die Reaktion mehrere Verfahrensschritte umfaßt, was weniger wirtschaftlich ist.

Aus US-A 5,420,354 ist ein Verfahren zur Herstellung von para-Phenylendiaminen bekannt, demgemäß Nitrobenzol mit Wasserstoff und Aminen in Gegenwart eines Hydrierkatalysators, eines Hydrierinhibitors und eines Säure-Cokatalysators in Kontakt gebracht werden, wobei die o. g. Phenylendiamine entstehen. Als Hydrierkatalysatoren werden dabei Metalle der 8. Gruppe des Periodensystems sowie Molybdän und Kupfer, gegebenenfalls auf Träger aufgebracht, eingesetzt. Als Hydrierinhibitoren werden schwefelhaltige organische Verbindungen eingesetzt. Auch dieses Verfahren ist mit Nachteilen behaftet und somit verbesserungswürdig.

Es war daher wünschenswert ein Verfahren zur Herstellung von 4-ADPA zur Verfügung zu stellen, das vom wohlfeilen Nitrobenzol ausgeht und in einem Reaktionsschritt in technisch brauchbaren Ausbeuten zum gewünschten 4-ADPA führt.

Gegenstand der vorliegenden Erfindung ist daher ein Verfahren zur Herstellung von 4-Aminodiphenylamin, das dadurch gekennzeichnet ist, daß man Nitrobenzol mit Wasserstoff in Gegenwart von hydroxid- und/oder oxidgruppenhaltigen Basen und bestimmten heterogenen Katalysatoren sowie in Gegenwart von inerten aprotischen Lösungsmitteln bei Temperaturen von 0 bis 200°C und Drücken von 0,1 bis 150 bar hydriert.

Als für das erfindungsgemäße Verfahren geeignete hydroxid- und/oder oxidgruppenhaltige Basen kommen in Frage anorganische Basen, wie Alkalimetallhydroxide, Alkalimetalloxide, Erdalkalimetallhydroxide, Erdalkalimetalloxide sowie die entsprechenden Hydroxide und Oxide der Elemente 58 bis 71 des Periodensystems der Elemente (nach IUPAC, neu). Beispielsweise werden genannt: Die Oxide und Hydroxide von Natrium, Kalium, Lithium, Caesium, Magnesium, Kalzium, Barium, Lanthan und/oder Cer, insbesondere die Oxide und Hydroxide von Lithium, Natrium, Kalium, Caesium, ganz besonders bevorzugt Caesiumhydroxid.

Weiterhin kommen organische Basen in Frage, wie beispielsweise quartäre Alkylammoniumhydroxide (NR₄⁺OH⁻ mit R unabhängig voneinander für Alkyl, Aryl oder Aralkyl mit 1 bis 7 Kohlenstoffatomen). Als Beispiele seien genannt: Tetramethylammoniumhydroxid, Tetraethylammoniumhydroxid, Tetrapropylammoniumhydroxid, Tetrabutylammoniumhydroxid, Methyltributylammoniumhydroxid, Methyltripropylammomumhydroxid, Methyltriethylammoniumhydroxid, Trimethylbenzylammoniumhydroxid. Besonders bevorzugt sind Tetramethylammoniumhydroxid, Tetraethylammoniumhydroxid, Tetrapropylammoniumhydroxid und Tetrabutylammoniumhydroxid. Ganz besonders bevorzugt wird Tetramethylammoniumhydroxid eingesetzt.

Selbstverständlich ist es auch möglich, die Basen in Mischungen untereinander einzusetzen. Das jeweils günstigste Mischungsverhältnis kann dabei leicht durch entsprechende Vorversuche ermittelt werden.

Weiterhin ist es möglich, die anorganischen Basen in Verbindung mit Phasentransferkatalysatoren einzusetzen. Geeignete Phasentransferkatalysatoren sind beispielsweise beschrieben in W.E. Keller, Fluka-Kompendium, Bd. 1, 2, 3, Georg Thieme Verlag, Stuttgart 1986, 1987, 1992. Beispielsweise können die zuvor erwähnten Basen mit Kronenether, wie 18-Krone-6 oder quartären Ammoniumverbindungen, zusammen eingesetzt werden.

Die erfindungsgemäß einzusetzenden Basen können einen Wassergehalt von bis zu 6 mol Wasser, bevorzugt bis zu 3 mol Wasser, besonders bevorzugt bis zu 2,5 mol Wasser, bezogen auf ein Mol Base, besitzen. Ein höherer Wassergehalt verschlechtert im allgemeinen die Ausbeuten.

Die erfindungsgemäßen Basen können dem Reaktionsgemisch in fester Form, als Schmelze oder als Lösung oder Gemisch, z.B. in Nitrobenzol oder in einem aprotischen Lösungsmittel oder in einer Mischung aus Nitrobenzol und einem oder mehreren aprotischen Lösungsmitteln zugesetzt werden.

Die Basen werden dabei in einer Menge von 0,01 bis 3, bevorzugt 0,1 bis 2, insbesondere 0,3 bis 1,5, Äquivalenten pro Mol Nitrobenzol eingesetzt.

Als inerte aprotische Lösungsmittel kommen aromatische Kohlenwasserstoffe mit 6 bis 20 Kohlenstoffatomen, lineare oder cyclische Ether mit bis zu 5 Sauerstoffatomen und 2 bis 16 Kohlenstoffatomen, aromatische halogenierte Kohlenwasserstoffe mit 6 bis 20 Kohlenstoffatomen sowie Amide mit 1 bis 10 Kohlenstoffatomen in Frage. Selbstverständlich können die erwähnten Lösungsmittel im Gemisch untereinander eingesetzt werden. Als geeignete Lösungsmittel werden insbesondere genannt: Benzol, Toluol, Xylol, tert.-Butylmethylether, tert.-Amylmethylether, Diisopropylether, Diethylenglykoldimethylether, Glycoldimethylether, Dioxan, Tetrahydrofuran, Diamylether, Chlorbenzol, Dichlorbenzol, Dimethylformamid, Dimethylacetamid und N-Methylpyrolidinon. Bevorzugt werden eingesetzt, Toluol, Xylol, Glykoldimethylether, tert.-Butylmethylether, Diisopropylether, Diethylenglykoldimethylether, insbesondere tert.-Butylmethylether und Toluol. Die Menge an Lösungsmittel ist für das erfindungsgemäße Verfahren nicht kritisch. Die geeignetste Menge kann ebenfalls leicht durch entsprechende Vorversuche ermittelt werden. Die Lösungsmittelmenge hängt insbesondere ab von der Reaktionstemperatur und von der Art und Menge der eingesetzten Basen und Katalysatoren. Üblicherweise werden die Lösungsmittel in Mengen von 1 bis 99 Gew.-%, bevorzugt 5 bis 95 Gew.-%, besonders bevorzugt 15 bis 90 Gew.-%, bezogen auf die Gesamtmenge der Reaktionsmischung, eingesetzt.

Für das erfindungsgemäße Verfahren eignen sich heterogene Katalysatoren auf der Basis von Metallen der 8-10 Gruppe des Periodensystems (nach IUPAC, neu) oder Kupfer und/oder Chrom auf geeignetem Träger mit einem Metallgehalt von 0,01 bis 50 Gew.-%, bevorzugt 0,1 bis 10 Gew.-%, bezogen auf das Gesamtgewicht des Katalysators. Erfindungsgemäß können Katalysatoren eingesetzt werden, die eines oder mehrere der obengenannten Metalle enthalten. Die angegebenen Gewichtsanteile gelten bei Anwesenheit mehrerer Elemente für die Summe der Einzelanteile. Bevorzugte Metalle sind insbesondere Platin, Palladium und Rhodium, besonders bevorzugt sind Platin und Palladium. Weitere bevorzugte Katalysatoren sind Raney-Nickel und geträgerte Nickelkatalysatoren.

Erfindungsgemäß können auch die obengenannten Metalle oder ihre Verbindungen in reiner Form als Feststoff eingesetzt werden. Als Beispiele für ein Metall in reiner Form seien Palladium- und Platinschwarz genannt.

Die Herstellung der erfindungsgemäßen Katalysatoren kann nach den verschiedensten Methoden erfolgen, die dem Fachmann bekannt sind. So können Lösungen einer oder mehrerer der genannten Metallverbindungen, beispielsweise durch Tränken, Adsorption, Tauchen, Sprühen, Imprägnieren und Ionenaustausch auf den erfindungsgemäß einzusetzenden Katalysatorträger gebracht werden. Dem Katalysator können in bekannter Art und Weise weitere Elemente zugefügt werden. Es ist weiterhin möglich, ein oder mehrere der genannten Metalle durch Fällung mit einer Base auf dem Träger zu fixieren. Als Base kommen z.B. (Erd-)Alkalimetallhydroxide in Frage. Ein oder mehrere Metalle können sowohl in beliebiger Reihenfolge nacheinander als auch gleichzeitig auf den Träger gebracht werden. Eine spezielle Ausführungsform der Erfindung beinhaltet das Aufbringen des Metalls durch Fällung eines Metallhalogenids oder einer Metallhalogenid-Komplexverbindung mit einer geeigneten Base und Reduktion der Metallverbindung zum Metall. Bei der Herstellung der Träger mittels eines Sol-Gel-Verfahrens können in einer Ausführungsform Lösungen einer oder mehrerer der genannten Metallverbindungen in dem Fachmann bekannter Weise bereits dem Sol zugesetzt werden.

Geeignete Materialien für die erfindungsgemäße Verwendung als Katalysatorträger sind alle technisch üblichen Katalysatorträger auf der Basis Kohlenstoff, Elementoxiden, Elementcarbiden oder Elementsalzen in verschiedenen Anwendungsformen. Beispiele für kohlenstoffhaltige Träger sind Koks, Graphit, Ruß oder Aktivkohlen. Beispiele für Elementoxid-Katalysatorträger sind SiO₂ (natürliche oder synthetische Kieselsäure, Quarz), Al₂O₃ (α-, γ-Al₂O₃), Tonerden, natürliche und synthetische Alumosilicate (Zeolithe), Schichtsilikate, wie Bentonit und Montmorillonit, TiO₂ (Rutil, Anatas), ZrO₂, MgO oder ZnO. Beispiele für Elementcarbide und -salze sind SiC, AlPO₄, BaSO₄, CaCO₃. Grundsäztlich können sowohl synthetische Materialien als auch Träger aus natürlichen Quellen, wie z.B. Bimsstein, Kaolin, Bleicherden, Bauxite, Bentonite, Kieselgur Asbest oder Zeolithe, verwendet werden.

Weitere brauchbare Träger für die erfindungsgemäß einsetzbaren Katalysatoren sind Elementmischoxide und Oxidhydrate von Elementen der Gruppen 2 bis 16 des Periodensystems sowie der Seltenerdmetalle (Atomnummern 58 bis 71), bevorzugt aus den Elementen Al, Si, Ti, Zr, Zn, Mg, Ca, Zn, Nb und Ce, die u.a. auf dem Weg über mechanische Vermischungen, gemeinsame Fällungen von Salzen oder über Cogele aus Salzen und/oder Alkoxiden hergestellt werden können, wie dies dem Fachmann bekannt ist.

Die Träger können sowohl im Sinne chemisch einheitlicher Reinsubstanzen als auch im Gemisch eingesetzt werden. Für die erfindungsgemäße Verwendung als Katalysatorträger eignen sich sowohl stückige als auch pulverförmige Materialien. Für den Fall der Anordnung des Träger-Katalysators als Festbett wird der Träger vorzugsweise als Formkörper, z.B. als Kugeln, Zylinder, Stäbchen, Hohlzylinder, Ringe usw. eingesetzt. Wahlweise können Katalysatorträger weiter durch Extrudieren, Tablettieren, gegebenenfalls unter Zumischen weiterer Katalysatorträger oder Bindemittel, wie SiO₂ oder Al₂O₃, und Kalzinieren modifiziert werden. Die innere Oberfläche der Träger (BET-Oberfläche) liegt bei 1 bis 2 000 m²/g, bevorzugt bei 10 bis 1 600 m²/g, ganz besonders bevorzugt bei 20 bis 1 500 m²/g. Darstellung und Weiterverarbeitung der erfindungsgemäß verwendeten Katalysatorträger sind dem Fachmann wohl bekannt und Stand der Technik.

Bevorzugt werden Aktivkohlen und Si-, Al-, Zr- und Ti-haltige Materialien als Trägermaterialien eingesetzt, besonders bevorzugt sind Aktivkohle sowie siliziumund aluminiumhaltige Träger.

Die erfindungsgemäßen Katalysatoren können in diskontinuierlichen Verfahrensvarianten in Mengen von 0,01 bis 20 Gew.-% bezogen auf eingesetztes Nitrobenzol verwendet werden, bevorzugt in Mengen von 0,01 bis 10 Gew.-%. Bei kontinuierlicher Durchführung der Reaktion, beispielsweise in einem Rührkessel mit einem pulverförmigen Katalysator oder in der Rieselphase am Festbettkatalysator, können Belastungen von 0,01 bis 500 g Nitrobenzol pro g Katalysator und Stunde verwendet werden. Bevorzugt sind Belastungen von 0,02 bis 300 g Nitrobenzol pro g Katalysator und Stunde.

Die Reaktionstemperaturen bei dem erfindungsgemäßen Verfahren betragen bevorzugt 0 bis 200°C, insbesondere 40 bis 150°C; die Drücke (Wasserstoffdruck) liegen bei 0,1 bis 150 bar, insbesondere 0,5 bis 70 bar, ganz besonders bevorzugt 1 bis 50 bar.

Es ist möglich, die Reaktion bei einer konstanten Temperatur und bei konstantem Wasserstoffdruck durchzuführen; Wasserstoffdruck und Temperatur können aber auch im Verlauf der Reaktion geändert werden bzw. in verschiedenen Reaktoren verschieden sein. Bei der diskontinuierlichen Durchführung können Nitrobenzol, Katalysator, Lösungsmittel und Base in beliebiger Reihenfolge in den Reaktor gefüllt werden. Die Wasserstoffzuführ kann nach einer bestimmten zugeführten Menge abgebrochen und gegebenenfalls später wieder fortgesetzt werden.

Kontinuierliche Verfahrensvarianten sind beispielsweise die Hydrierung in der Sumpfphase mit einem pulverförmigen suspendierten Katalysator (Slurry), die Hydrierung in der Rieselphase am Festbett-Katalysator oder die Hydrierung mit einem suspendierten Katalysator in einer Blasensäule. Die Reaktion kann in den dem Fachmann bekannten Apparaten zur Kontaktierung von Fest-, Flüssig- und Gasphasen durchgeführt werden. Insbesondere kommen hier Rührkessel, Umpumpreaktoren, Busreaktoren, im Gleich- oder Gegenstrom betriebene Blasensäulen oder Rieselphasenreaktoren oder Kaskaden dieser Reaktoren, in Frage, wobei die verschiedenen Reaktortypen auch gleichzeitig in einer Kaskade vorkommen können.

Wird der Katalysator als Pulver in der Sumpfphase eingesetzt, sind zur Vermischung der Reaktionskomponenten, die zu verwendenden Rührbehälter mit dafür brauchbaren Rührern ausgestattet. Möglich sind der Einsatz von Flügel-, MIG-, Propeller-, Ankeroder Begasungsrührer.

Es ist besonders überraschend, daß man bei dem erfindungsgemäßen Verfahren ausgehend von Nitrobenzol in einer katalytischen Hydrierreaktion in technisch guten Ausbeuten (>20 % der Theorie) 4-Aminodiphenylamin herstellen kann. Dies überrascht um so mehr, da es bisher nicht gelang, durch katalytische Hydrierung von Nitrobenzol 4-Aminodiphenylamin in merklichen Mengen zu erhalten. Es ist nämlich bekannt, daß bei der katalytischen Hydrierung von Nitrobenzol insbesondere Anilin, Azoxybenzol, Azobenzol sowie Hydrazobenzol erhalten werden (siehe z.B. Ullmann 5th Ed. Vol. A2, 1985, S. 303-311; S.C. Karwa, Ind. Eng. Chem. Res. 27, 22 (1988); J. Wisniak, Ind. Eng. Chem. Res. 1984, 23, 44-50; US 5 420 354). Erst mit Hilfe des erfindungsgemäßen Verfahrens gelingt es, wie bereits ausgeführt, gezielt 4-Aminodiphenylamin in technisch interessanten Ausbeuten zu erhalten.

Darüber hinaus bei dem erfindungsgemäßen Verfahren anfallende Stoffe sind Zwischenprodukte der Hydrierung von Nitrobenzol zu Anilin und lassen sich restlos in Anilin überführen, welches ebenfalls ein wertvolles Ausgangsprodukt für die Synthese vieler industrieller Endprodukte ist.

### Beispiele

In den folgenden Beispielen wurden die Reaktionsprodukte gaschromatographisch (Permabond SE-52-DF-0,25; 25m x 0,32mm ID) mit dem internen Standard Methylstearat bzw. mittels quantitativer HPLC analysiert. Der Umsatz an Nitrobenzol war in allen beschriebenen Versuchen vollständig. Aufarbeitung und Probenpräparation wurde unter Stickstoff durchgeführt. Die angegebenen Hydrierdrücke im Autoklav wurden bei reaktionsbedingtem Druckabfall von Hand nachgeregelt.

Die Tetramethylammoniumhydroxid-Hydrate (TMAOH ^{·} xH₂O) wurden wie folgt hergestellt: Handelsübliches Pentahydrat (Fa. Aldrich) oder 25 % wässrige Lösung (Fa. Aldrich) wurden bei 80°C im Wasserstrahlvakuum und danach im Pumpenvakuum oder im Exsiccator über Phosphorpentoxid Wasser entzogen. Der erreichte Wassergehalt wurde durch Säure-Titration festgestellt. Der Wassergehalt des jeweils eingesetzten Materials ist in den Ansätzen spezifiziert.

### Herstellung der Katalysatoren A, B, C

### Katalysator A (Pt-Katalysatoren)

Das Trägermaterial wurde 2 h bei 350°C im Muffelofen ausgeheizt und in einen 250 ml Kolben überführt. Am Rotationsverdampfer wurde zum Trägermaterial eine verdünnte wäßrigen Lösung von H₂PtCl₆ gegeben (Durch Verdünnen einer wäßrigen H₂PtCl₆-Lösung mit 25 % Platingehalt hergestellt). Die Wassermenge war so gewählt worden, daß sie komplett vom Träger aufgenommen wurde ("incipient wetnet"). Anschließend wurde der größte Teil des Wassers bei 60°C im Vakuum abdestilliert, der Katalysator 16 h bei 120°C bei einem Druck <1 mbar getrocknet und in einem Strom von 10 Vol-% Wasserstoff in Stickstoff für 24 h bei 300°C reduziert. Der Pt-Gehalt wurde mittels Elementaranalyse bestimmt.

| Bsp.Nr | Typ | Träger | BET m²/g | Träger (g) | "Pt" | "H₂O" | "Pt%" |
|---|---|---|---|---|---|---|---|
| A1 | SiO₂ | Grace Type 432 | 320 | 50,0 | 2,02 | 90,0 | 0,84 |
| A2 | TiO₂ | Degussa P25 | 50 | 30,8 | 1,22 | 93,5 | 1,04 |
| A3 | ZrO₂ | Degussa VP ZrO₂ | 40 | 28,8 | 1,19 | 60,7 | 1,05 |
| A4 | Al₂O₃ | Rhone-Poulenc SPH509 | 335 | 30,0 | 1,23 | 71,0 | 0,96 |
| AS | Al₂O₃/SiO₂ (1,2% Al) | Grace Typ III/10 | 345 | 30,2 | 1,23 | 86,4 | 1,03 |
| A6 | SiO₂/ZrO₂ (3,9% Zr) | Grace SP2-8402.01 | 306 | 50,0 | 2,08 | 158,0 | 0,98 |
| "Pt": Menge an H₂PtCl₆-Lösung (25% Pt-Gehalt); "H₂O": Menge an zugegebenem Wasser; Pt%: Platingehalt des fertigen Katalysators in Gew.-%. | | | | | | | |

### Katalysator B (Pt/Aktivkohle)

475 g Aktivkohle (Norit-B-Supra, Fa. Norit) wurden in 2 600 ml entionisiertem Wasser aufgeschlämmt, die Mischung auf 50°C erwärmt und mit einer Lösung von 87,5 g Natriumformiat in 400 ml entionisiertem Wasser versetzt. In 30 Minuten wurde eine Mischung aus 100 g einer H₂PtCl₆-Lösung (25 Gew.-% Pt) und 400 ml entionisiertem Wasser zugetropft und eine Stunde bei 50°C nachgerührt. Anschließend wurde der Katalysator abgesaugt, gewaschen und im Vakuum getrocknet.

### Katalysator C (Rh/Pd/Pt-Katalysator)

100 g γ-Al₂O₃-Kugeln (SPH501, Rhone Poulenc) wurden mit einer Lösung aus 0,421 g PdCl₂, 0,674 g RhCl₃ x 4 H₂O und 2,02 g einer wäßrigen H₂PtCl₆-Lösung (Pt-Gehalt 25 %) in 2,66 g konz. HCl und 36,3 g destilliertem H₂O getränkt, bis die Flüssigkeit vollständig aufgesaugt war. Anschließend wurde der Katalysator 5 Minuten bei 50°C im Wirbelstrom getrocknet, und 24 h bei 375°C in einem Strom von 10 Vol-% Wasserstoff in Stickstoff reduziert.

### A) Vergleichbeispiele:

Die Vergleichsbeispiele demonstrieren, daß Umsetzungen in protischen Lösungsmitteln nicht in nennenswerten Maße zu 4-ADPA führen:

### Vergleichsbeispiel 1: Umsetzung in alkoholischer Lösung:

960 ml Ethanol, 63.6 g TMAOH^{.}2.0 H₂O, 132,0 g Nitrobenzol (1.1 mol) und 4,8 g Pt/C - Katalysator B wurden in dem stickstoffgespülten Autoklaven vorgelegt. Unter Rühren und Kühlen wurden 5 bar Wasserstoff aufgedrückt und 3 h 45 min hydriert. Anschließend wurde das Reaktionsgemisch filtriert und bei max. 70°C und leichtem Vakuum das Ethanol am Rotationsverdampfer abgezogen.

Das eingeengte Reaktionsgemisch wurde in einem Scheidetricher mit 500 ml Toluol versetzt und dreimal mit 1000 ml dest. Wasser extrahiert. Die organische Phase wurde im Vakuum einrotiert und analysiert. Gehalt an 4-ADPA: <1 g.

### Vergleichsbeispiel 2: Umsetzung in wässriger Lösung

Eine Lösung von 90 g Tetramethylammoniumhydroxid (1 mol TMAOH) in 960 ml Wasser wurde zusammen mit 132,0 g Nitrobenzol (1,1 mol) und 4,8 g Pt/C (Katalysator B) in einem stickstoffgespülten Autoklaven vorgelegt. Unter Rühren und bei 80°C wurde mit 4 bar Wasserstoff 370 min hydriert. Anschließend wurde die Mischung auf 50°C abgekühlt, unter Stickstoff filtriert und mit 1 000 ml Toluol versetzt. Das Gemisch wurde in einen Scheidetrichter überführt, geschüttelt und die Phasen getrennt. Nach der Phasentrennung wurde die organische Phase noch zweimal mit je 1 000 ml Wasser extrahiert. Die organische Phase wurde bei max. 70°C im Vakuum einrotiert. Der Rückstand wurde ausgewogen und analysiert: Er enthielt kein 4-ADPA.

### B) Lösungsmitteleinfluß

Folgende Versuche demonstrieren die Vielfalt der einsetzbaren aprotischen Lösungsmittel

### Beispiel 1 (tert.-Butylmethylether)

960 ml tert.-Butylmethylether, 127,5 g TMAOH^{.}2.0 H₂O (1,0 mol), 132 g Nitrobenzol (1,1 mol) und 2,4 g Pd/C - Kat. (5 % Pd/Powder Carbon S-95-386, E169 Mallinckrodt) wurden in einem stickstoffgespülten Autoklaven vorgelegt. Unter Rühren wurde bei 80°C mit 5 bar Wasserstoff 9 h hydriert. Dann wurde auf 50°C abgekühlt und das Reaktionsgemisch unter Stickstoff filtriert. Ebenso wurde mit dem Waschwasser, mit dem der Autoklav ausgespült worden war, verfahren. Die Filtrate wurden in einem Scheidetrichter vereinigt und geschüttelt. Nach der Phasentrennung wurde die organische Phase noch zweimal mit je 1 000 ml dest. Wasser extrahiert. Das Lösungsmittel wurde abdestilliert und der Rückstand einer Vakuumdestillation unterzogen. Die Fraktion, die bei 11 bis 12 mbar und 160 bis 166°C überging, enthielt 23,1 g 4-ADPA (Selektivität: 22,8 %).

### Beispiel 2 (Toluol)

960 ml Toluol, 127,0 g, TMAOH^{.}2.15 H₂O (0,98 mol), 132,0 g Nitrobenzol (1,1 mol) und 4,8 g Pt/C-Katalysator B wurden in einem stickstoffgespülten Autoklaven vorgelegt. Unter Rühren wurde bei 80°C mit 5 bar Wasserstoff 2 h hydriert. Anschließend wurde auf 50°C abgekühlt. Das Reaktionsgemisch wurde unter Stickstoff filtriert. Ebenso wurde mit dem Waschwasser (ca. 2 000 ml), mit dem der Autoklav ausgespült worden war, verfahren. Die Filtrate wurden in einem Scheidetrichter vereinigt, 500 ml Toluol und NaCl zugesetzt und geschüttelt. Die organische Phase wurde noch zweimal mit je 1 000 ml dest. Wasser extrahiert und bei max. 70°C im Vakuum einrotiert. Der Rückstand wurde gemörsert und bei einem Vakuum <1 mbar destilliert. Bei 136°C bis 157°C destillierte ein Produkt über. Nach GC -Analytik enthielt das Destillat 11.5 g 4-ADPA (Selektivität: 11,4 %).

### Beispiel 3 (tert.-Butylmethylether)

960 ml tert.-Butylmethylether, 128,0 g TMAOH^{.}2.15 H₂O (0,98 mol), 132,0 g Nitrobenzol (1,1 mol) 4,8 g Pt/C-Katalysator B wurden in dem stickstoffgespülten Autoklav vorgelegt. Unter Rühren (800 U/min.) wird 4 h 40 min mit 5 bar Wasserstoff bei 80°C hydriert. Anschließend wurde das Reaktionsgemisch auf 50°C abgekühlt und unter Stickstoff filtriert. Ebenso wurde mit dem Waschwasser (ca. 2 000 ml), mit dem der Autoklav ausgespült worden war, verfahren. Die Filtrate wurden in einem Scheidetrichter vereinigt und geschüttelt. Die organische Phase wurde noch zweimal mit je 1 000 ml dest. Wasser extrahiert. Die erste wässrige Phase wurde mit 500 ml Toluol extrahiert. Die organischen Phasen wurden vereinigt und mittels GC analysiert. Ausbeute: 15,8 g 4-ADPA (Selektivität: 15,6 %).

### Beispiel 4 (Diisopropylether)

960 ml Diisopropylether, 125,4 g TMAOH^{.}1.9 H₂O (1,0 mol), 132,0 g Nitrobenzol (1,1 mol) und 4,8 g Pd/C - Kat. (5 % Pd/C-Katalysator aus Beispiel 1) wurden in einem stickstoffgespülten Autoklaven vorgelegt und auf 80°C temperiert. Unter Rühren (800 U/min.) wird bei 80°C 10,5 h mit 5 bar Wasserstoff bei 80°C hydriert. Anschließend wurde auf 50°C abgekühlt. Das Gemisch wurde mit 2 l Wasser und ca. 800 ml Toluol aus den Autoklaven herausgelöst und unter Stickstoff abfiltriert. Die vereinigten Filtrate wurden anschließend im Scheidetrichter geschüttelt. Die organische Phase wurde noch zweimal mit jeweils 1 000 ml dest. Wasser extrahiert. Die organische Phase wurde mittels GC und HPLC analysiert. Ausbeute: 17,8 g 4-ADPA (Selektivität: 17,6 %).

### Beispiel 5 ( tert.-Butylmethylether/Toluol)

960 ml tert.-Butylmethylether/Toluol, 1:1, (V:V) 127,0 g TMAOH^{.}2.0 H₂O (1,0 mol), 132,0 g Nitrobenzol (1,1 mol) und 2,4g Pd/C - Kat. (5% Pd/C-Katalysator aus Beispiel 1) wurden in dem stickstoffgespülten Autoklav vorgelegt. Mit 5 bar Wasserstoff wurde 5 h 15 min bei 80°C hydriert. Anschließend wurde das Reaktionsgemisch auf 50°C abgekühlt und unter Stickstoffüberlagerung filtriert. Ebenso wurde mit 2 l Waschwasser, mit dem der Autoklav ausgespült worden war, verfahren. Die Filtrate wurden vereinigt und in einem Scheidetrichter geschüttelt. Nach der Phasentrennung wurde die organische Phase noch zweimal mit je 1 000 ml dest. Wasser extrahiert. Die organische Phase wurde mittels GC und HPLC analysiert. Ausbeute: 16,0 g 4-ADPA (Selektivität: 15,9 %).

### Beispiel 6 (Diglyme)

960 ml Diethylenglykoldimethylether, 127,5 g TMAOH 2.0 H₂O (1,0 mol), 132,0 g Nitrobenzol (1,1 mol) und 2,4g Pd/C - Katalysator (5% Pd/C-Katalysator aus Beispiel 1), wurden in einem stickstoffgespülten Autoklaven vorgelegt. Bei 80°C wird 3 h 40 min mit 5 bar Wasserstoff hydriert. Das Reaktionsgemisch und 2 l Waschwasser, mit dem der Autoklav ausgespült worden war, wurden vereinigt, mit 1 000 ml Toluol versetzt, geschüttelt und unter Stickstoff filtriert. Das Filtrat wurde in einem Scheidetrichter geschüttelt. Nach der Phasentrennung wurde die organische Phase noch zweimal mit je 1 000 ml dest.Wasser extrahiert. Die organische Phase wurde mittels GC und HPLC analysiert. Ausbeute: 12,1 g 4-ADPA (Selektivität: 11,9 %).

### Beispiel 7 (tert.-Amylmethylether)

960 ml tert.-Amylmethylether, 127,5 g TMAOH^{.}2.0 H₂O (1,0 mol), 132,0 g Nitrobenzol (1,1 mol) und 2,4 g Pd/C - Kat. (5 % Pd/C-Katalysator aus Beispiel 1), wurden in einem stickstoffgespülten Autoklaven vorgelegt. Bei 80°C wird 8 h 30 min mit 5 bar Wasserstoff hydriert. Das Reaktionsgemisch wurde unter Stickstoff filtriert. Ebenso wurde mit dem dem Waschwasser, mit dem der Autoklav ausgespült worden war, verfahren. Die Filtrate wurden in einem Scheidetrichter vereinigt und geschüttelt. Die abgetrennte organische Phase wurde noch zweimal mit je 1 000 ml dest. Wasser extrahiert. Die organische Phase wurde mittels GC und HPLC analysiert. Ausbeute: 14,2 g 4-ADPA (Selektivität: 14,0 %).

### C) Basenvariation

Folgende Versuche demonstrieren, daß verschiedene Basenmengen mit verschiedenen Hydratmengen geeignet sind.

### Beispiel 8 (1 mol TMAOH-Dihydrat)

960 ml tert.-Butylmethylether, 127,5 g TMAOH^{.}2.0 H₂O (1,0 mol), 132 g Nitrobenzol (1,1 mol), 2,4 g Pd/C - Kat. (5 % Pd/C-Katalysator aus Beispiel 1), wurden in einem stickstoffgespülten Autoklaven vorgelegt. Bei 80°C wird 7 h 10 min mit 5 bar Wasserstoff hydriert. Das Reaktionsgemisch wurde unter Stickstoff filtriert. Ebenso wurde mit dem Waschwasser, mit dem der Autoklav ausgespült worden war, verfahren. Die Filtrate wurden in einem Scheidetrichter vereinigt und geschüttelt. Nach der Phasentrennung wurde die organische Phase noch zweimal mit je 1 000 ml dest. Wasser extrahiert. Die organische Phase wurde mittels GC und HPLC analysiert. Ausbeute: 17,2g 4-ADPA (Selektivität: 17,0 %).

### Beispiel 9 (½ Mol TMAOH-Dihydrat)

960 ml tert.-Butylmethylether, 63,5 g TMAOH^{.}2.0 H₂O (0,5 mol), 132 Nitrobenzol (1,1 mol) und 2,4 g Pd/C - Kat. (5% Pd/C-Katalysator aus Beispiel 1), wurden in einem stickstoffgespülten Autoklaven vorgelegt. Bei 80°C wird 6 h mit 5 bar Wasserstoff hydriert. Das Reaktionsgemisch wurde unter Stickstoff filtriert. Ebenso wurde mit dem Waschwasser, mit dem der Autoklav ausgespült worden war, verfahren. Die Filtrate wurden vereinigt und in einem Scheidetrichter geschüttelt. Nach der Phasentrennung wurde die organische Phase noch zweimal mit je 1 000 ml dest. Wasser extrahiert. Die organische Phase wurde mittels GC und HPLC analysiert. Ausbeute: 10,5 g 4-ADPA (Selektivität: 10,4 %).

### Beispiel 10 (2 Mol TMAOH-Dihydrat)

960 ml tert.-Butylmethylether, 255,0 g TMAOH^{.}2.0 H₂O (2,0 mol Base), 132,0 g Nitrobenzol (1,1 mol) und 2,4 g Pd/C - Kat. (5 % Pd/C-Katalysator aus Beispiel 1) wurden in einem stickstoffgespülten Autoklaven vorgelegt. Bei 80°C wird 7 h 30 min mit 5 bar Wasserstoff hydriert. Im Reaktionsgemisch enthaltener ungelöster Feststoff wurde mit 1 000 ml Wasser und 300 ml Toluol gelöst. Dann wurde die Lösung ebenso wie das Waschwasser, mit dem der Autoklav ausgespült worden war, unter Stickstoff filtriert. Das Filtrat wurde in einem Scheidetrichter geschüttelt. Nach der Phasentrennung wurde die organische Phase noch zweimal mit je 1 000 ml dest. Wasser extrahiert. Die organische Phase wurde mittels GC und HPLC analysiert. Ausbeute: 18,07 g 4-ADPA (Selektivität: 17,8 %).

### Beispiel 11 (1 Mol TMAOH-Dihydrat + 1 Mol Wasser)

960 ml tert.-Butylmethylether, 127,5 g TMAOH^{.}2.0 H₂O (1,0 mol), 132 g Nitrobenzol (1,1 mol), 18 g dest. Wasser und 2,4 g Pd/C - Katalysator (5 % Pd/C-Katalysator aus Beispiel 1) wurden in einem stickstoffgespülten Autoklaven vorgelegt. Unter Rühren wurden 5 bar Wasserstoff aufgedrückt. Bei 80°C wird 10 h 30 min mit 5 bar Wasserstoff hydriert. Das Reaktionsgemisch wurde unter Stickstoff filtriert. Ebenso wurde mit dem Waschwasser, mit dem der Autoklav ausgespült worden war, verfahren. Die Filtrate wurden vereinigt und in einem Scheidetrichter geschüttelt. Nach der Phasentrennung wurde die organische Phase noch zweimal mit je 1 000 ml dest. Wasser extrahiert. Die organische Phase wurde mittels GC und HPLC analysiert. Ausbeute: 10,4 g 4-ADPA (Selektivität: 10,3 %).

### Beispiel 12 (1 Mol TMAOH-1.5 H₂O)

960 ml tert.-Butylmethylether, 118,2 g TMAOH^{.}1.5 H₂O (1,0 mol), 132 g Nitrobenzol (1,1 mol) und 2,4 g Pd/C - Kat. (5 % Pd/C-Katalysator aus Beispiel 1), wurden in einem stickstoffgespülten Autoklaven vorgelegt. Unter Rühren wurden 5 bar Wasserstoff aufgedrückt. Bei 80°C wird 6 h 30 min mit 5 bar Wasserstoff hydriert. Das Reaktionsgemisch wurde unter Stickstoff filtriert. Ebenso wurde mit dem Waschwasser, mit dem der Autoklav ausgespült worden war, verfahren. Die Filtrate wurden vereinigt und in einem Scheidetrichter geschüttelt. Nach der Phasentrennung wurde die organische Phase noch zweimal mit je 1 000 ml dest. Wasser extrahiert. Die organische Phase wurde mittels GC und HPLC analysiert.
Ausbeute: 15.0 g 4-ADPA (Selektivität: 14,8 %).

### D) Höhere Konzentrationen:

### Beispiel 13

480 ml tert.-Butylmethylether, 127,5 g TMAOH^{.}1.5 H₂O (1,0 mol), 132,0 g Nitrobenzol (1,1 mol) und 4,8 g Pd/C - Katalysator (5 % Pd/C-Katalysator aus Beispiel 1), wurden in einem stickstoffgespülten Autoklaven vorgelegt. Unter Rühren wurden 5 bar Wasserstoff aufgedrückt. Bei 80°C wird 4 h 45 min mit 5 bar Wasserstoff hydriert. Das Reaktionsgemisch wurde unter Stickstoff filtriert. Ebenso wurde mit dem Waschwasser, mit dem der Autoklav ausgespült worden war, verfahren. Die Filtrate wurden vereinigt und in einem Scheidetrichter geschüttelt. Nach der Phasentrennung wurde die organische Phase noch zweimal mit je 1 000 ml dest. Wasser extrahiert. Die organische Phase wurde mittels GC und HPLC analysiert.
Ausbeute: 18.0 g 4-ADPA (Selektivität: 17,8 %).

### E) Temperatur- und Druckvariation

Folgende Beispiele demonstrieren benutzbare Temperaturen und Drücke.

### Beispiel 14 (60°C)

820 ml Toluol, 110 g TMAOH^{.}2.1 H₂O (0,85 mol), 113 g Nitrobenzol (0,92 mol) und 4,1 g Pt/C - Katalysator B wurden in einem stickstoffgespülten Autoklaven vorgelegt. Unter Rühren wurden 6 h mit 5 bar Wasserstoff hydriert. Anschließend wurde das Reaktionsgemisch auf 50°C abgekühlt und unter Stickstoff filtriert. Ebenso wurde mit dem Waschwasser, mit dem der Autoklav ausgespült worden war, verfahren. Die Filtrate wurden in einem Scheidetrichter vereinigt und geschüttelt. Nach der Phasentrennung wurde die organische Phase noch zweimal mit je 1 000 ml dest. Wasser extrahiert. Die organische Phasen wurden vereinigt und mittels GC analysiert.
Ausbeute: 8,2 g 4-ADPA (Selektivität: 8,1 %).

### Beispiel 15 (100°C)

960 ml Toluol, 128,9 g TMAOH^{.}2.1 H₂O (1,0 mol), 132,0 g Nitrobenzol (1,1 mol) und 4,8 g Pt/C - Katalysator B wurden in einem stickstoffgespülten Autoklaven vorgelegt. Bei 100°C wird 1 h 55 min mit 5 bar Wasserstoff hydriert. Das Reaktionsgemisch wurde gemeinsam mit dem Waschwasser unter Stickstoff filtriert, mit 500 ml Toluol versetzt und im Scheidetrichter geschüttelt. Die organische Phase wurde noch zweimal mit jeweils 1 000 ml dest. Wasser extrahiert. Die organische Phase wurde mittels GC und HPLC analysiert.
Ausbeute: 14,4 g 4-ADPA (Selektivität: 14,2 %).

### Beispiel 16: (100°C)

960 ml tert.-Butylmethylether, 127,5 g TMAOH^{.}2.0 H₂O (1,0 mol), 132,0 g Nitrobenzol (1,1 mol) und 2,4 g Pd/C - Kat. (5 % Pd/C-Katalysator aus Beispiel 1), wurden in einem stickstoffgespülten Autoklaven vorgelegt. Bei 80°C wurde 5 h mit 5 bar Wasserstoff hydriert. Der Autoklav wurde mit 2 1 Wasser gereinigt und das Reaktionsgemisch wurde mit dem Waschwasser und 200 ml Toluol filtriert unter Stickstoff. Die separierte organische Phase wurde noch zweimal mit jeweils 1 000 ml dest. Wasser extrahiert. Die organische Phase wurde mittels GC und HPLC analysiert.
Ausbeute: 16,8 g 4-ADPA (Selektivität: 16,6 %).

### Beispiel 17: (10 atm)

960 ml tert.-Butylmethylether, 127,5 g TMAOH^{.}2.0 H₂O (1,0 mol), 132,0 g Nitrobenzol (1,1 mol) und 1,2 g Pd/C - Kat. (5 % Pd/C-Katalysator aus Beispiel 1) wurden in einem stickstoffgespülten Autoklaven vorgelegt. Bei 80°C wurde 12,5 h mit 5 bar Wasserstoff hydriert. Der Autoklav wurde mit 2 l Wasser gereinigt und das Reaktionsgemisch wurde mit dem Waschwasser und 200 ml Toluol filtriert unter Stickstoff. Die separierte organische Phase wurde noch zweimal mit jeweils 1 000 ml dest. Wasser extrahiert. Die organische Phase wurde mittels GC und HPLC analysiert.
Ausbeute: 16,8 g 4-ADPA (Selektivität: 16,6 %).

### Beispiel 18: (50 atm) Dieser Versuch demonstriert ebenfalls, daß auch hohe Konzentrationen geeignet sind

246 g tert.- Butylmethylether, 250,7 g TMAOH^{.}1.9 H₂O (2,0 mol), 246,0 g Nitrobenzol und 4,8 g Pd/C - Kat. (5 % Pd/C-Katalysator aus Beispiel 1), wurden stickstoffgespülten Autoklaven vorgelegt. Bei 80°C wurde 24 h mit 50 bar Wasserstoff hydriert. Nach Zugabe von 400 ml Toluol wurde der Ansatz zusammen mit dem Waschwasser, mit dem der Autoklav ausgespült worden war, unter Stickstoff filtriert. Die Filtrate wurden in einem Scheidetrichter vereinigt und geschüttelt. Die organische Phase wurde noch zweimal mit je 1 000 ml dest. Wasser extrahiert und mittels GC analysiert.
Ausbeute: 28,8 g 4-ADPA (Selektivität: 14,2 %).

### F) Variation der Katalysatoren

Folgende Beispiele demonstrieren die Verwendung verschiedener Katalysatoren und Katalysatormengen. Darüber hinaus demonstrieren sie die Durchführbarkeit der Reaktion bei Normaldruck.

### Beispiele 19 - 35 (Normaldruckhydrierungen)

In einem mit Stickstoff gespülten 250 ml Planschlifftopf mit Begasungsrührer wurden 75 ml Diethylenglykoldimethylether oder 48 g Toluol, 7,03 g TMAOH^{.}2.0 H₂O, und der pulverförmige Katalysator vorgelegt und auf 80°C erwärmt. Nach Erreichen dieser Temperatur wurde der Stickstoff bei Normaldruck durch einen Wasserstoffstrom von 25 l/h ersetzt und gleichzeitig 6,77 g Nitrobenzol zugegeben. Nach 120 min wurde eine Probe entnommen, filtriert, mit Essigsäure neutralisiert und mittels quantitativer Gaschromatografie analysiert. Der Umsatz an Nitrobenzol war in allen Versuchen vollständig. Die Ergebnisse sind in untenstehender Tabelle wiedergegeben.

| Bsp. | Katalysator | Katalysator Menge [g] | LM | S |
|---|---|---|---|---|
| 19 | 5% Pd/C, Engelhard, Code 3230 Lot 2942 | 0,2 | T | 25,8 |
| 20 | 5% Pd/C, Engelhard, Code 3230 Lot 2942 | 0,1 | T | 23,8 |
| 21 | 5% Pd/C (Aldrich) | 0,5 | D | 14,8 |
| 22 | 10% Pd/C (Aldrich) | 0,25 | D | 12,2 |
| 23 | 5% Pt/C, Mallinkrodt, S-95-38b, E169 | 0,5 | D | 18,1 |
| 24 | Katalysator B | 0,25 | D | 24,7 |
| 25 | Katalysator B | 0,5 | T | 17,6 |
| 26 | 5% Rh/Al₂O₃ (Aldrich) | 0,5 | D | 17,0 |
| 27 | 5% Pd/BaSO₄ (Aldrich) | 0,5 | D | 17,8 |
| 28 | 5% Pd/BaCO₃ (Aldrich) | 0,5 | D | 14,4 |
| 29 | Katalysator A1 | 2.3 | D | 22,4 |
| 30 | Katalysator A2 | 2,4 | D | 19,1 |
| 31 | Katalysator A3 | 2,6 | D | 21,8 |
| 32 | Katalysator A4 | 2,6 | D | 23,6 |
| 33 | Katalysator A5 | 2,4 | D | 16,7 |
| 34 | Katalysator A6 | 1,0 | D | 23,5 |
| 35 | Katalysator C | 2,1 | D | 20,8 |
| LM: Lösungsmittel, D: Diglyme, T: Toluol, S: Selektivität zu 4-ADPA in Mol% bez. auf Nitrobenzol. | | | | |

### Beispiel 36 (Rhodium/C-Katalysator, Druckreaktion)

960 ml tert. Butyl-methylether, 127,5 g TMAOH^{.}2.0 H₂O (1,0 mol), 132 g Nitrobenzol (1,1 mol) und 2,4 g (5 % Rh/C, Merck; Best. Nr. 818 851), wurden in einem stickstoffgespülten Autoklaven vorgelegt. Bei 80°C wird 6 mit 5 bar Wasserstoff hydriert. Das Reaktionsgemisch wurde unter Stickstoff filtriert. Ebenso wurde mit dem Waschwasser, mit dem der Autoklav ausgespült worden war, verfahren. Die Filtrate wurden in einem Scheidetrichter vereinigt und geschüttelt. Nach der Phasentrennung wurde die organische Phase noch zweimal mit je 1 000 ml dest. Wasser extrahiert. Die organische Phase wurde mittels GC und HPLC analysiert.
Ausbeute: 13,6 g 4-ADPA (Selektivität: 13,4 %).

## Patentansprüche

1. Verfahren zur Herstellung von 4-Aminodiphenylamin, **dadurch gekennzeichnet, daß** man Nitrobenzol mit Wasserstoff in Gegenwart von hydroxidund/oder oxidgruppenhaltigen Basen und heterogenen Katalysatoren auf Basis von Metallen der 8. - 10. Gruppen des Periodensystems oder Kupfer und/oder Chrom auf geeignetem Träger mit einem Metallgehalt von 0,01 - 50 Gew.-%, bezogen auf das Gesamtgewicht des Katalysators, sowie in Anwesenheit von inerten aprotischen Lösungsmitteln bei Temperaturen von 0 bis 200°C und Drücken von 0,1 bis 150 bar hydriert.

2. Verfahren nach Anspruch 1, **dadurch gekennzeichnet, daß** man bei Temperaturen von 40 bis 150°C und Drücken von 0,5 bis 70 bar hydriert.

3. Verfahren nach Anspruch 1, **dadurch gekennzeichnet, daß** man als hydroxidund/oder oxidgruppenhaltige Basen Alkalihydroxide, Erdalkalimetalloxide, Erdalkalimetallhydroxide, Erdalkalimetalloxide, die entsprechen Hydroxide und Oxide der Elemente 58 bis 71 des Periodensystems der Elemente (IUPAC, neu) sowie quartäre Alkylammoniumhydroxide einsetzt.

4. Verfahren nach Anspruch 1, **dadurch gekennzeichnet, daß** man die Basen in Mengen von 0,01 bis 3 Äquivalente pro Mol Nitrobenzol einsetzt.

5. Verfahren nach Anspruch 1, **dadurch gekennzeichnet, daß** man als aprotische Lösungsmittel aromatische Kohlenwasserstoffe mit 6 bis 20 Kohlenstoffatomen, lineare oder cyclische Ether mit bis zu 5 Sauerstoffatomen und 2 bis 16 Kohlenstoffatomen, aromatische halogenierte Kohlenwasserstoffe mit 6 bis 20 Kohlenstoffatome, sowie Amide mit 1 bis 10 Kohlenstoffatomen einsetzt.

6. Verfahren nach Anspruch 1, **dadurch gekennzeichnet, daß** man die inerten aprotischen Lösungsmittel in Mengen von 1 bis 99 Gew.-%, bezogen auf die Gesamtmenge der Reaktionsmischung, einsetzt.

7. Verfahren nach Anspruch 1, **dadurch gekennzeichnet, daß** man den Katalysator in diskontinuierlichen Verfahrensvarianten in Mengen von 0,01 bis 20 Gew.-% bezogen auf eingesetztes Nitrobenzol verwendet oder in kontinuierlichen Verfahrensvarianten Belastungen von 0,01 bis 500g Nitrobenzol pro g Katalysator und Stunde verwendet.

## Claims

1. Process for the production of 4-aminodiphenylamine, **characterised in that** nitrobenzene is hydrogenated with hydrogen in the presence of bases containing hydroxide and/or oxide groups and heterogeneous catalysts which are based on metals of groups 8 to 10 of the periodic system or copper and/or chromium on a suitable support and have a metal content of 0.01-50 wt.%, relative to the total weight of the catalyst, and in the presence of inert aprotic solvents at temperatures of 0 to 200°C and pressures of 0.1 to 150 bars.

2. Process according to Claim 1, **characterised in that** hydrogenation is performed at temperatures of 40 to 150°C and pressures of 0.5 to 70 bars.

3. Process according to Claim 1, **characterised in that** alkali metal hydroxides, alkaline earth metal oxides, alkaline earth metal hydroxides, alkaline earth metal oxides, the corresponding hydroxides and oxides of elements 58 to 71 of the periodic system of elements (IUPAC, new) and quaternary alkylammonium hydroxides are used as bases containing hydroxide and/or oxide groups.

4. Process according to Claim 1, **characterised in that** the bases are used in quantities of 0.01 to 3 equivalents per mol of nitrobenzene.

5. Process according to Claim 1, **characterised in that** aromatic hydrocarbons with 6 to 20 carbon atoms, linear or cyclic ethers with up to 5 oxygen atoms and 2 to 16 carbon atoms, aromatic halogenated hydrocarbons with 6 to 20 carbon atoms, and amides with 1 to 10 carbon atoms are used as aprotic solvents.

6. Process according to Claim 1, **characterised in that** the inert aprotic solvents are used in quantities of 1 to 99 wt.%, relative to the total quantity of the reaction mixture.

7. Process according to Claim 1, **characterised in that**, in discontinuous process variants, the catalyst is used in quantities of 0.01 to 20 wt.%, relative to the nitrobenzene used, or, in continuous process variants, loadings of 0.01 to 500 g of nitrobenzene per g of catalyst per hour are used.

## Revendications

1. Procédé de préparation de la 4-aminodiphénylamine, **caractérisé en ce que** l'on effectue l'hydrogénation du nitrobenzène avec de l'hydrogène en présence de bases contenant des radicaux hydroxyde et/ou oxyde et de catalyseurs hétérogènes à base des métaux des groupes 8-10 du système périodique ou du cuivre et/ou du chrome sur un support approprié, avec une teneur en métal allant de 0,01 à 50% en poids, sur base du poids total de catalyseur, ainsi qu'en présence de solvants inertes aprotiques à des températures allant de 0 à 200°C et des pressions allant de 0,1 à 150 bar.

2. Procédé suivant la revendication 1, **caractérisé en ce que** l'on réalise l'hydrogénation à des températures allant de 40 à 150°C et des pressions allant de 0,5 à 70 bar.

3. Procédé suivant la revendication 1, **caractérisé en ce que** l'on met en oeuvre comme base contenant des radicaux hydroxyde et/ou oxyde, des hydroxydes alcalins, des oxydes de métal alcalin, des hydroxydes de métal alcalino-terreux, des oxydes de métal alcalino-terreux, les hydroxydes et oxydes des éléments 58 à 71 du système périodique des éléments (IUPAC, nouveau), ainsi que des hydroxydes d'alcoylammonium quaternaire.

4. Procédé suivant la revendication 1, **caractérisé en ce que** l'on met en oeuvre la base en des quantités allant de 0,01 à 3 équivalents par mole de nitrobenzène.

5. Procédé suivant la revendication 1, **caractérisé en ce que** l'on met en oeuvre comme solvant aprotique, des hydrocarbures aromatiques ayant 6 à 20 atomes de carbone, des éthers linéaires ou cycliques ayant jusqu'à 5 atomes d'oxygène et 2 à 16 atomes de carbone, des hydrocarbures aromatiques halogénés ayant 6 à 20 atomes de carbone, ainsi que des amides ayant 1 à 10 atomes de carbone.

6. Procédé suivant la revendication 1, **caractérisé en ce que** l'on met en oeuvre le solvant inerte aprotique en des quantités allant de 1 à 99% en poids, sur base de la quantité totale du mélange réactionnel.

7. Procédé suivant la revendication 1, **caractérisé en ce que** l'on utilise le catalyseur, dans les variantes discontinues du procédé, en des quantités allant de 0,01 à 20% en poids, sur base du nitrobenzène mis en oeuvre ou on utilise, dans les variantes continues du procédé, des charges allant de 0,01 à 500 g de nitrobenzène par g de catalyseur et par heure.
